Europäisches Patentamt

European Patent Office

Office européen des brevets

(19)

(11) Publication number: **0 382 334**
**A2**

(12) **EUROPEAN PATENT APPLICATION**

(21) Application number: **90300122.0**

(22) Date of filing: **05.01.90**

(51) Int. Cl.⁵: **G01N 33/82**

(30) Priority: **09.02.89 US 308862**

(43) Date of publication of application:
**16.08.90 Bulletin 90/33**

(84) Designated Contracting States:
**DE FR GB IT**

(71) Applicant: **BIO-RAD LABORATORIES, INC.**
**1000 Alfred Nobel Drive**
**Hercules California 94547(US)**

(72) Inventor: **Murthy, Shashidhara H.M.**
**288 Shipley Avenue**
**Daly City California 94015(US)**
Inventor: **Tun Zan, Geraldine**
**315 Alberta Way**
**Hillsborough California 94010(US)**

(74) Representative: **Harrison, David Christopher**
**et al**
**MEWBURN ELLIS 2 Cursitor Street**
**London EC4A 1BQ(GB)**

(54) **Microwave method for preparation of serum samples for b-12 and folate assays.**

(57) The present invention provides novel processes for the release of vitamin $B_{12}$ and/or folate in mammalian serum samples from endogenous serum binders comprising microwaving the serum samples prior to assaying the serum samples for vitamin $B_{12}$ and/or folate. Prior to the assay procedure, the serum samples are typically microwaved for about 20-90 seconds, most preferably for about 60 seconds at a high setting or such that the temperature of the serum samples is brought to a temperature of about 65 to 100° C, most preferably 80-85° C, for at least 15 seconds.

EP 0 382 334 A2

# MICROWAVE METHOD FOR PREPARATION OF SERUM SAMPLES FOR B-12 AND FOLATE ASSAYS

## BACKGROUND OF THE INVENTION

Field of the Invention

This invention relates to the release of vitamin $B_{12}$ (cobalamin) and/or folate (folic acid) from endogenous binders, and more particularly to the preparation of mammalian serum for assays for vitamin $B_{12}$ and/or folate.

The assay of vitamin $B_{12}$ in humans is a valuable technique for diagnosing and subsequently treating certain diseases which cause a deficiency of vitamin $B_{12}$, such as for example, pernicious anemia, post gastrectomy states, nutritional deficiencies, intestinal disorders, and other conditions where the blood level of vitamin $B_{12}$ is frequently depressed. The assay of vitamin $B_{12}$ in humans is also useful for diagnosing certain myeloproliferative diseases, such as chronic myelogenous leukemia where the blood level of vitamin $B_{12}$ may be elevated.

Initially vitamin $B_{12}$ was assayed, for example, using either Euglena gracilis or Lactobacillus leichmannii in microbiological assays. More recently, radioisotope dilution (RID) assays for vitamin $B_{12}$ have been utilized. Such RID assay techniques for vitamin $B_{12}$ are well-known and well-documented in the literature. See, for example, Lau, et al. "Measurement of Serum $B_{12}$ Levels Using Radioisotope Dilution and Coated Charcoal," BLOOD, 26: 202-204 (1965), as modified by Raven, et al. "Improved Method for Measuring Vitamin $B_{12}$ in Serum Using Intrinsic Factor, $^{57}$Co-$B_{12}$ and Coated Charcoal," JOURNAL OF CLINICAL PATHOLOGY, 22:205 (1969), both of which are incorporated by reference herein.

The presence of vitamin $B_{12}$ analogues in samples may falsely contribute to the apparent vitamin $B_{12}$ level found by RID assay, unless the binding protein utilized in the assay is specific to vitamin $B_{12}$, as for example, either pure intrinsic factor, or binding protein which has been treated to eliminate or inactivate binding proteins which are non-specific to vitamin $B_{12}$, Kolhouse, et al., "Cobalamin analogues present in human serum and can mask Cobalamin deficiency...." N.E.J. MED. 229: 784 (1978).

The $B_{12}$ and its analogues are bound to endogenous carrier proteins in serum. These proteins typically are heptocorrin and transcobalamin I and II which have an affinity constant of $10 \times 10^{-8}$ for $B_{12}$ and its analogues. Current RID assay of vitamin $B_{12}$ generally requires a step for the liberation of the endogenous vitamin $B_{12}$ from its endogenous natural binding protein by, for example, boiling the serum sample to be tested according to the teaching of Kolhouse, et al. The boiling methods to date utilize boiling water baths where the serum samples need to be kept at a high temperature for relatively long periods of time -- usually by submission in the boiling water bath for at least 15-20 minutes.

Following the inactivation of endogenous binding proteins, a known amount of intrinsic factor (specific for $B_{12}$) is added. Radioactive vitamin $B_{12}$, for example, $^{57}$Co-$B_{12}$, is also added in a known amount sufficient by itself to bind all of the intrinsic factor. Thus, all of the added intrinsic factor should be bound, either by the vitamin $B_{12}$ present in the serum sample or by the radioactive vitamin $B_{12}$ added to the composition. As both the natural vitamin $B_{12}$ and the radioactive vitamin $B_{12}$ compete dynamically in the reaction to bind with the limited amount of intrinsic factor binding protein, the degree to which the radioactive count of the intrinsic factor-bound vitamin $B_{12}$ is inhibited or reduced is indicative of the amount of natural vitamin $B_{12}$ which is present in the sample undergoing testing. The inhibition of the binding of the radioactive vitamin $B_{12}$ with intrinsic factor is typically determined by separating all unbound vitamin $B_{12}$ from the intrinsic factor-bound vitamin $B_{12}$ by using, for example, protein-coated charcoal which adsorbs the unbound radioactive vitamin $B_{12}$ or by the use of bentonite as described in Lewin, et al., U.S. Patent No. 3,937,799, which is incorporated by reference herein. The radioactivity of the supernatant liquid containing the mixture of intrinsic factor-bound radioactive vitamin $B_{12}$ is then counted for radioactivity. The vitamin $B_{12}$ concentration in the serum sample is then determined from the count, often by comparison with a standard curve using techniques well known in the art.

Folate RID analysis is quite similar using radioactive folate and a folate binder·in the same manner. There are no similar problems with folate analogues in the folate assay, however, and no similar problems with non-specific folate binder. Both folate and vitamin $B_{12}$ competitive assay techniques require the heating or boiling of samples prior to testing, in order to liberate vitamin $B_{12}$ and/or folate from endogenous binding protein in the serum samples to be assayed. The current boiling step is difficult to control, tedious and time consuming.

Means of denaturing samples chemically, to liberate vitamin $B_{12}$ from endogenous binding protein without boiling, have also been reported. Typically, these methods involve the addition of 2N sodium hydroxide to the sample and after twenty minutes, the alkali is neutralized with the addition of a buffered tracer. See, for example, PCT publication No. WO82/03461, Application No. PCT/US82/00426. The use of organic liquids such as acetone has also been described, to denature the proteins to release $B_{12}$. See, for example, Mansbach, et al., U.S. Patent No. 4,300,907. In each of these chemical techniques KCN is normally added for the primary purpose of converting the vitamin $B_{12}$ in the sample to its most stable form, cyanocobalamin. See, for example, PCT publication No. WO82/03461, Application No. PCT/US82/00426. Similarly, dithiothreitol is normally used in folate assays, and therefore in dual vitamin $B_{12}$-folate assays, as a means of stabilizing folate during the folate assay. See, for example, Lewin, et al., U.S. Patent No. 4,028,465. These chemical methods are time consuming as well and still require the use of toxic chemicals. Thus, there is a need for a method of denaturation of serum proteins which is quicker and simpler than the boiling method and which is safer than the chemical methods.

## SUMMARY OF THE INVENTION

The methods of the present invention are simpler, quicker, safer and more commercially practical than previously known methods for denaturation of serum proteins. The denaturation process of the present invention which utilizes any standard microwave oven may be done neatly, safely, completely and quickly in about one minute.

The microwave method of the present invention is a surprisingly effective method for denaturation of proteins such that vitamin $B_{12}$ and/or folate are released from endogenous serum binders. The method further enables the denaturation to occur with minimal disturbance to the integrity of the resident vitamin $B_{12}$ or folate.

The present invention provides novel processes for the release of vitamin $B_{12}$ and/or folate in mammalian serum samples from endogenous serum binders comprising microwaving the serum samples prior to assaying the serum samples for vitamin $B_{12}$ and/or folate. The serum samples are typically microwaved for about 20-90 seconds, preferably for about 30-60 seconds, most preferably for about 60 seconds at a high setting.

## BRIEF DESCRIPTION OF THE DRAWINGS

Fig. 1 shows the non-linear increase in release of bound vitamin $B_{12}$ in a human serum sample over time when subjected to microwaving at a high setting (closed circles) and the lack of release of bound vitamin $B_{12}$ over time in samples which were not microwaved (open circles).

Fig. 2 is a plot of a standard curve for use in the present assay where serum samples are microwaved for 60 seconds at a high setting. Values obtained from unknown serum can be applied to the curve to find serum vitamin $B_{12}$ concentrations.

## DETAILED DESCRIPTION OF THE INVENTION

Preparation of mammalian serum samples for assay of vitamin $B_{12}$ or folate requires the release of vitamin $B_{12}$ and/or folate from endogenous binders. The methods of the present invention provide for a simple, quick, complete and clean method for releasing vitamin $B_{12}$ or folate by the use of a conventional microwave oven.

Placement of serum samples to be assayed for vitamin $B_{12}$ or folate may be microwaved by placing them in a conventional microwave oven and subjecting them to microwaves for a sufficient period of time to allow for the release of vitamin $B_{12}$ or folate from endogenous serum binders. The release of endogenous serum binders is complete when the serum binders capable of binding vitamin $B_{12}$ and/or folate are irreversibly denatured. Measurement of the release of $B_{12}$ or folate may be tested by any of the methods well-known in the art, one such method being more specifically set forth in the Example below. For instance, given the guidelines set forth herein, those skilled in the art may readily determine the optimum time and temperature settings in a particular microwave oven for the release of $B_{12}$ or folate according to the methods of the present invention.

Any conventional microwave oven which dispenses microwaves may be used. Microwave ovens typically have settings of low, medium, high and gradations between those. For the purposes of this invention, a high setting refers to one where the magnetron of the oven dispenses microwaves 80%-100% of the time that the oven is operating. A medium-high setting refers to one where the magnetron dispenses microwaves 60%-80% of the time the oven is on. At a medium setting microwaves are dispensed 40%-60% of the time, at a low setting microwaves are dispensed 40% of the time or less. Typically, if the microwave power levels are designated 1-10, the level 10 corresponds with a setting where the magnetron dispenses microwaves 100% of the time, level 9 corresponds with a setting where the magnetron dispenses microwaves 90% of the time, etc.

For the methods of this invention it is preferred that microwaves capable of generating high wattage such as those generating 700 watts be used. Further, it is preferred that high power levels be used and that the samples to be assayed are microwaved for about 20-90 seconds, preferably for about 30-60 seconds, most preferably for about 60 seconds. It is contemplated that the power level and time period used will vary with the type of oven selected, as well as with the size and number of samples. Further, if medium-high power levels or lower are chosen, a longer time period for microwaving may be desirable.

Alternatively, the serum samples are microwaved to bring the serum samples to a temperature of about 65 to about 100°C, preferably about 80-100°C, most preferably about 80 to about 85°C for a minimum of about 15-20 seconds. The serum samples may be microwaved at those temperatures for up to 60 seconds.

Preferably the size of an individual serum sample is at least 150 µl. Typically the serum samples are about 150-250 µl in size, most preferably about 200 µl in size.

The methods of the present invention may be used to prepare mammalian serum samples for any conventional $B_{12}$ and/or folate assay where it is desirable to release the vitamin $B_{12}$ and/or folate from endogenous binders. These methods are particularly useful for competitive binding assay techniques such as RID, which techniques are well-known in the field. Examples of such techniques can be found in U.S. Patent No. 3,937,799, Lewin, et al., U.S. Patent No. 4,028,465, Cabelli, et al., U.S. Patent No. 4,332,786, all of which are incorporated by reference herein.

The microwave methods of the present invention are surprisingly complete methods for denaturation of the proteins which enable the denaturation to occur with minimal disturbance to the integrity of the resident vitamin $B_{12}$ or folate. Past heating methods utilized boiling water baths where the serum needed to be heated for long, continuous periods of time, typically 15-20 minutes. Since the samples needed to be maintained at the boiling temperature for 15-20 minutes in the prior methods, it was surprising that the high power of the microwave at a very short period of time of 60 seconds or less would be effective. It was further surprising that lower power levels were not as effective, even over time.

Examples

1. Microwave:

In all the tests a Sharp Carousel II type microwave oven (Sharp Electronics Corp., Mahwah, N.J.) capable of an output of 700 watts was used.

2. Denaturation of Serum at High Settings:

$^{57}$Co labelled B-12 was manufactured based on the procedure and principles of saturation analysis described in Ekins, R. P. Clin. Chim. Acta. 5, 453 (1960) and Lau, K.S., et al., Blood, 26, 202 (1965), both incorporated by reference herein, with the following modifications:

Viable cultures of Pseudomonas shermanii (A.T.C.C. #13673) in isolated colonies were transferred to B-12 liquid media in Stage I at 30°C for 30 minutes and a second suspended culture from Stage I to Stage II liquid media at 30°C for 30 minutes cultured with the best growth were chosen for fermentation purposes. A calculated volume of cobalt chloride (57°C) was spiked into the Stage II B-12 liquid media and the fermentation process was continued for four (4) days in a dark room. Fermentation growth was spiked with 5-6-dimethyl benzimidzole for another 24 hours and the cell suspension was subjected to column purification:

1. ALA columns AG 1 x 8 AC 100-200 mesh (Bio-Rad Catalog #187-2000, Richmond, CA)

4

2. Column with SM-2 Bio-Beads (20-50 mesh) (Bio-Rad Catalog #152-3920, Richmond, CA)

The $^{57}$Co labelled B-12 was eluted from the columns using acetic acid and methanol and further purified by a resin column (cation exchange) in a 20 x 1 AG 50 W-X2(100-200 mesh) Na$^+$ form and concentration diluted to 10 $\mu$Ci/ml in 0.9% benzyl alcohol.

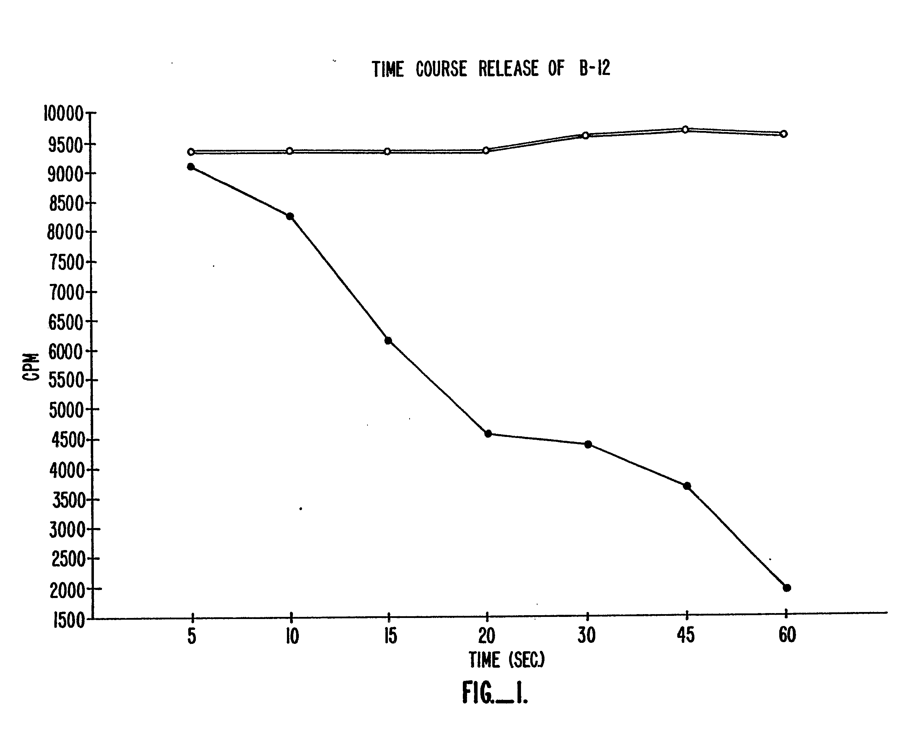

In each test tube was placed 200 $\mu$l of a human serum sample. One ml of $B_{12}$ tracer, $^{57}$Co labeled $B_{12}$, was added to each tube and then each tube was mixed well and incubated at room temperature for 10 minutes to allow the labeled $B_{12}$ to bind to the serum binding proteins. Half of the tubes were maintained as controls and were not microwaved. The other half were microwaved at a high setting. Separate sets of tubes were microwaved for 5, 10, 15, 20, 30, 45 or 60 seconds. After each time interval, tube samples were allowed to cool to room temperature and measured for the release of $B_{12}$ by the charcoal method described in Lau, et al., and Raven, et al., both supra. Charcoal tablets were added to each tube to bind the free $B_{12}$. The tubes were then incubated for five minutes and then vortexed. Each tube was centrifuged at 3000 RPM for ten minutes. The $B_{12}$, bound and remaining in the supernatant, was measured with a gamma counter. Thus, $B_{12}$ released by microwaving became bound to the activated charcoal and the amount of labeled $B_{12}$ remaining in the supernatant represented that which remained bound to the serum binding proteins. The results can be seen on Table I and Fig. 1. The samples exposed to the microwaves contained more released bound $B_{12}$, while those samples not microwaved contained little or no released $B_{12}$. In addition, the data indicates that the amount of bound $B_{12}$ released increased with the amount of time the samples were exposed to the microwaves, although Fig. 1 demonstrates that the release of bound $B_{12}$ was non-linear.

TABLE I

| Microwave at High Setting | | |
| --- | --- | --- |
| TIME | $B_{12}$ CTS MICROWAVED | $B_{12}$ CTS NO MICROWAVE |
| 5 SEC | 9095 | 9356 |
| 10 SEC | 8252 | - |
| 15 SEC | 6172 | - |
| 20 SEC | 4690 | - |
| 30 SEC | 4409 | 9593 |
| 45 SEC | 3708 | 9695 |
| 60 SEC | 1968 | 9619 |
| Total Counts | 15234 | |

3. Denaturation of Serum at Med-High and Medium Setting:

In a second test some serum sample tubes prepared as above were microwaved at a medium setting and another set was microwaved at a medium-high setting for 10, 20, 30, 45 or 60 seconds. At the different time intervals, a tube set was taken out and cooled. Bound and free $B_{12}$ were separated using the charcoal method above. The data obtained from this experiment is set forth on Table II. The results indicate an incomplete release of bound $B_{12}$. At 60 seconds, some $B_{12}$ was still bound to serum proteins.

TABLE II

| Microwave at Med-High or Medium Setting | | |
|---|---|---|
| TIME | $B_{12}$ CTS MED. HIGH | $B_{12}$ CTS MED. |
| 10 SEC | 7327 | 8739 |
| 20 SEC | 7795 | 8524 |
| 30 SEC | 4886 | 6319 |
| 45 SEC | 3801 | 5868 |
| 60 SEC | 4507 | 5409 |
| Total Counts | 15540 | |

### 4. $B_{12}$ Assay Using the Microwave Method:

Test tubes (12x75 mm) were labeled for standards, blank control and serum sample. To the tubes were added 200 $\mu$l of standards, control or patient serum sample as appropriate. To the blank tubes were added 200 $\mu$l of zero standard. To all tubes was further added 1ml of $^{57}$Co labeled $B_{12}$ tracer. The tubes were microwaved for 60 seconds at the high setting and then cooled to room temperature. An aliquot of 100 $\mu$l of microbead reagent comprising intrinsic factor and folate binding proteins coupled to the microbead reagent as described below was added to each tube. To the blank tubes was added 100 $\mu$l of blank reagent. All tubes were vortexed and then incubated at room temperature for one hour. After incubation the tubes were centrifuged for 10 minutes, decanted and counted with a gamma counter. Controls used were obtained from Environmental Chemical Specialties, Anaheim, CA and Gilford, Ciba-Corning Diagnostics, Oberlin, Ohio.

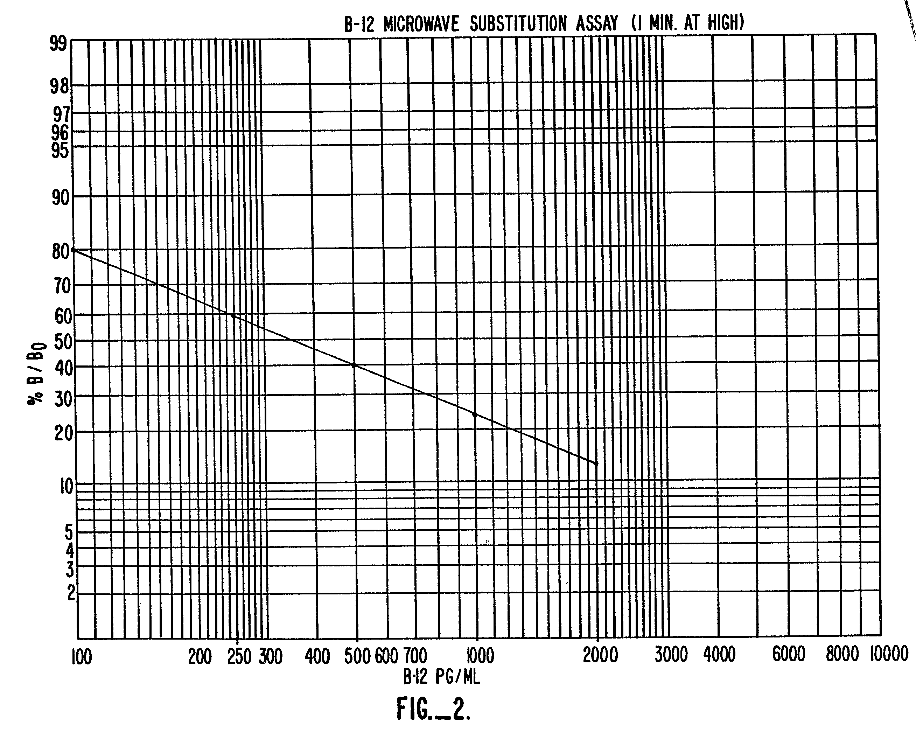

Table III sets forth the resulting counts for the standards containing known concentrations of $B_{12}$. The percentage of bound $B_{12}$ decreases with the increased concentration of unlabeled $B_{12}$ in the standards. An almost complete displacement of labeled $B_{12}$ occurs at the higher concentrations of $B_{12}$ in the standard. The data represented on a log-logit graph yields a straight line. Fig. 2 represents the standard curve. The data on the patient sample is represented in Table IV. Table IV also includes data regarding the release of folate using the microwave method. Folate is released under the microwave method to a degree similar to the boiling method.

TABLE III

| STANDARDS ($B_{12}$) | % BOUND $B_{12}$ |
|---|---|
| Total Counts (CPM) | 16022.00 |
| BLANK (CPM) | 739.00 |
| ZERO STD | 100.00 |
| 100 pg/ml | 79.60 |
| 250 pg/ml | 58.50 |
| 500 pg/ml | 39.60 |
| 1000 pg/ml | 23.60 |
| 2000 pg/ml | 12.99 |

The microbead reagent consisted of a mixture of intrinsic factor and folate binding proteins coupled to microbeads (6-20 $\mu$ size polyacrylamide beads, Bio-Rad Laboratories). Purified folate binding proteins were dialyzed in deionized water and coupled to hydrated beads at pH 6.3 using EDAC (Ethyl-3(3-dimethylamino propyl carbodiimide)) overnight. The coupled beads were washed with low salt and high salt buffers and resuspended to a concentration of 10 mg/ml.

The purified intrinsic factor was dialyzed overnight against deionized water and coupled to hydrolyzed

6

beads at pH 4.9 using EDAC. The beads were washed with high salt and low salt buffer and concentration was adjusted to 10g/L. The microbead reagent mixture contained 10 mg/ml of folate binding protein coupled beads and 10 mg/ml of intrinsic factor coupled beads. The exact suspension of beads were titered and matched with reference beads.

TABLE IV

| B-12/FOLATE BOILING METHOD (BOTH) VS MICROWAVE ASSAY | | | | |
|---|---|---|---|---|
| | BOIL FOLATE | BOIL B-12 | MICROWAV 1 MIN @ FOLATE | MICROWAV 1 MIN @ B-12 |
| NB | 3571.800 | 714.70 | 3531.000 | 739.59 |
| TC | 84488.300 | 16050.00 | 84805.000 | 16022.00 |
| BO | 41622.000 | 11675.90 | 45027.000 | 11310.40 |
| NSB | 4.228 | 4.45 | 4.160 | 4.62 |
| UC | 40.230 | 60.36 | 43.500 | 57.40 |
| TR. BIND. | 49.300 | 72.70 | 53.090 | 70.60 |
| SLOPE | -1.104 | -1.16 | -1.017 | -1.09 |
| INT. | 1.220 | 6.82 | 0.980 | 6.38 |
| R | -0.990 | -0.99 | -0.990 | -0.99 |
| ED 50 | 3.040 | 349.00 | 2.620 | 344.00 |
| CONTROLS | | | | |
| E1 17501 | 1.940 | 523.83 | 1.990 | 603.64 |
| E2 17502 | 5.530 | 667.60 | 5.670 | 758.50 |
| E3 17503 | 15.270 | 1063.87 | 16.690 | 1160.83 |
| ORTHO 16BO | 12.440 | 830.83 | 13.160 | 908.33 |
| ORTHO 17B0 | 4.800 | 555.40 | 4.950 | 591.50 |
| ORTHO 18BO | 1.720 | 144.60 | 1.790 | 155.00 |
| PATIENT SA | | | | |
| #1 | 17.030 | 40.14 | 17.190 | 94.77 |
| #2 | 3.630 | 100.20 | 3.840 | 252.61 |
| #3 | 1.420 | 971.90 | 1.360 | 1159.30 |
| #4 | 5.840 | 71.40 | 6.000 | 140.11 |
| #5 | 5.320 | 195.23 | 5.240 | 228.60 |
| #6 | 3.250 | 100.75 | 3.260 | 176.68 |
| #7 | 4.290 | 228.80 | 4.610 | 279.48 |
| #8 | 5.150 | 127.00 | 5.080 | 197.20 |
| #9 | 2.130 | 238.70 | 2.150 | 352.32 |
| #10 | 2.250 | 262.90 | 2.180 | 315.47 |
| #11 | 4.010 | 137.70 | 4.100 | 138.60 |
| #12 | 2.540 | 1993.13 | 1.950 | 2082.90 |
| #13 | 0.600 | 369.46 | 0.830 | 526.97 |
| #14 | 3.650 | 116.50 | 4.280 | 240.00 |
| #15 | 7.690 | 72.42 | 7.690 | 117.88 |
| #16 | 0.390 | 180.22 | 0.490 | 315.43 |
| #17 | 8.510 | 149.65 | 9.410 | 173.34 |
| #18 | 1.740 | 765.90 | 1.820 | 902.37 |
| #19 | 14.220 | 131.13 | 18.240 | 271.46 |
| #20 | 1.750 | 845.15 | 1.590 | 1058.07 |

FOLATE E1 = 1.95 RANGE 1.57-2.33

E2 = 5.79 RANGE 5.31-6.27

E3 = 15.41 RANGE 12.63-18.19

B-12 E1 = 548 RANGE 502-594

E2 = 664 RANGE 590-738

E3 = 1018 RANGE 878-1158

With the use of a dual tracer such as $^{125}$I folate plus $^{57}$Co-$B_{12}$, both folic acid and $B_{12}$ concentrations can be assayed in the same sample. These assays are well-known and practiced in the art.

Although the present invention has been described in some detail by way of illustration and example for purposes of clarity and understanding, the illustrations and examples should not be construed as a limitation upon the claims.

## Claims

1. A process for the release of vitamin $B_{12}$ in mammalian serum samples from endogenous serum binders comprising microwaving the serum samples prior to assaying the serum samples for vitamin $B_{12}$.

2. The process of claim 1, wherein the serum samples are microwaved for about 20-90 seconds.

3. The process of claim 1, wherein the serum samples are microwaved at a medium-high to high setting.

4. The process of claim 1, wherein the serum samples are human serum samples.

5. The process of claim 1, wherein the serum samples are microwaved to bring the samples to a temperature of about 65 to about 100° C for at least 15 seconds.

6. The process of claim 1, wherein the serum samples are microwaved to bring the samples to a temperature of about 80 to about 85° C for at least 15 seconds.

7. A process for the release of folate in mammalian serum samples from endogenous serum binders comprising microwaving the serum samples prior to assaying the serum samples for folate.

8. The process of claim 7, wherein the serum samples are microwaved for about 20-90 seconds.

9. The process of claim 7, wherein the serum samples are microwaved at a medium-high to high setting.

10. The process of claim 7, wherein the serum samples are human serum samples

11. The process of claim 7, wherein the serum samples are microwaved to bring the samples to a temperature of about 65 to about 100° C for at least 15 seconds.

12. The process of claim 7, wherein the serum samples are microwaved to bring the samples to a temperature of about 80 to about 85° C for at least 15 seconds.

13. A process for the release of vitamin $B_{12}$ and/or folate in mammalian serum samples from endogenous serum binders comprising microwaving the serum samples to bring the samples to a temperature of about 80 to about 85° C for at least 15 seconds prior to assaying the serum samples for vitamin $B_{12}$ and/or folate.

TIME COURSE RELEASE OF B-12

FIG._I.

EP 0 382 334 A2

# B-12 MICROWAVE SUBSTITUTION ASSAY (1 MIN. AT HIGH)

FIG._2.